# EUROPEAN PATENT SPECIFICATION

(11) **EP 0 687 911 B1**
(45) Date of publication and mention of the grant of the patent: **17.10.2001**
(21) Application number: 95201582.4
(22) Date of filing: 21.06.1989
(51) Int. Cl.: G01N 33/579

(54) **Methods for assaying endotoxin concentration**
Verfahren zumm Assay von Endotoxin-Konzentration
Méthodes pour l'essai de la concentration d'endotoxine

(30) Priority: 23.06.1988 US 210575
(43) Date of publication of application: 20.12.1995
(62) Divisional of application: 89907603.8
(73) Proprietor: ASSOCIATES OF CAPE COD, INC., Falmouth, MA 02540 (US)
(72) Inventor: Wainwright, Norman R., Massachusetts 02540 (US); Novitsky, Thomas J., Massachusetts 02540 (US)
(74) Representative: Chapman, Paul William

(56) References cited:
- EP-A- 0 056 210
- EP-A- 0 224 830

## Description

### Technical Field

The present invention is directed to assays for endotoxin, particularly assays using an endotoxin binding protein isolated from a horseshoe crab.

### Background Art

Endotoxins are high molecular weight complexes, associated with the outer membrane or gram-negative bacteria, that produce pyrogenic reactions upon intravenous administration. Endotoxin is shed from living bacteria and is also released into the environment when bacteria die and decompose. Since gram-negative bacteria are found in great numbers in air, water, and soil, bacterial endotoxin commonly contaminates raw materials and processing equipment used in the manufacturing of, for example, pharmaceuticals.

Bacterial endotoxin is a complex consisting of lipid, carbohydrate and protein. It is characterized by an overall negative charge, heat stability and high molecular weight. Highly purified endotoxin does not contain protein, and is a lipopolysaccharide (LPS). Depyrogenation can generally be achieved by inactivating or removing endotoxin, depending upon the physicochemical nature of the LPS. LPS consists of three distinct chemical regions, lipid A, which is the innermost region, an intermediate core polysaccharide, and an outermost O-specific polysaccharide side chain which is responsible for an endotoxin's particular immunospecificity.

Bacterial endotoxins are known to have profound biological effects in animals and humans, and to cause severe medical problems when present. Symptoms include induction of high fever, activation of complement, and hypotension. It is critical to avoid endotoxin contamination in any pharmaceutical product or medical device which comes into contact with body fluids. High endotoxin levels in sera due to bacterial diseases, such as septicemia, are not easily treated. Antibiotic treatment of the infection only kills the bacteria, leaving the endotoxin from their cell walls free to cause fever.

The horseshoe crab Limulus polyphemus is particularly sensitive to endotoxin. The cells from their hemolymph (amebocytes) undergo a complex series of biochemical reactions resulting in clot formation, analogous to mammalian blood coagulation. This phenomenon has been exploited in the form of bioassays sensitive to very low endotoxin levels. Currently, a bioassay of this type is the method of choice for monitoring pharmaceutical manufacturing and is termed Limulus Amebocyte Lysate (LAL). See U.S. Patents 4,276,050, 4,273,557, 4,221,866, 4,201,865, 4,038,147, 3,944,391 and 3,915,805, each of which is incorporated herein by reference.

It has long been observed that once endotoxin interacts with LAL the toxin is not recoverable from the clot. See Nachum et al, Journal of Invertebrate Pathology, 32:51-58 (1978). This observation lead investigators to postulate two alternative explanations. Either the endotoxin is enzymatically degraded during clot formation or it is bound by some factor causing it to lose toxicity. The present inventors initiated experiments to extract the endotoxin inactivating factor from the LAL.

Other research groups have experimented with endotoxin binding proteins, also referred to as anti-LPS factor. To the inventor's knowledge, the following publications resulting from work in this area are the most relevant to this invention:
Tanaka et al, Biochem. Biophys. Res. Comm. 105, 717-723 (1982),
Iwanaga et al, International Symposium on Pyrogen, 84-84 (June 23-26, 1987),
Aketagawa et al, J. Biol. Chem. 261, 7354-7365 (1986),
Hao, U.S. Patent 4,677,194 (June 30, 1987), and
Nachum et al, J. Inv. Path. 32, 51-58 (1978).
Tanaka et al, Iwanaga et al, and Aketagawa et al each conducted research on an anti-LPS factor or endotoxin binding protein isolated from a horseshoe crab system. Based on experimental work done in the inventors' laboratory, it appears that a protein involved in the present invention is the same as that isolated by Iwanaga et al and Tanaka et al. However, these publications do not say anything about pharmaceutical utility of the endotoxin binding protein, and it is difficult to predict in vivo activity based on in vitro experimentation. In fact, neither or these references suggests a practical utility for the anti-LPS factor, and in view of the unpredictable nature of in vivo activity, it has previously not been appreciated that the endotoxin binding protein could be used in a pharmaceutical composition. Furthermore, none of the references disclose the use of the endotoxin binding protein for an endotoxin assay, as disclosed in the present invention. It is notable that the present inventors have also discovered certain endotoxin binding protein variants which have amino acid structures that are different from the anti-LPS factor disclosed in the above-described publications.

### Disclosure of Invention

It is an object of the present invention to provide a method of assaying for endotoxin in a fluid. Thus, the present invention provides
a method for assaying endotoxin concentration, which comprises contacting serial aqueous dilutions of material suspected of containing endotoxin with a known quantity of endotoxin binding protein, observing the fluorescence emission at at least one wavelength of the endotoxin binding protein before and after contact with aqueous dilutions of said material suspected of containing endotoxin,
correlating the levels of fluorescence emission with at least one known emission level to thereby determine the quantity of endotoxin present in said material suspected of containing endotoxin.

These and other objects of the present invention which will hereinafter become more readily apparent, have been provided by purifying an endotoxin binding protein from the horseshoe crab Limulus polyphemus, and discovering that it has the capability of binding to endotoxin in vivo. The present inventors have also discovered that there are certain structural variants of the Limulus polyphemus endotoxin binding protein, and it is expected that these materials will also possess endotoxin binding ability such that they may be used in the other aspects of the present invention as well.

### Brief Description of the Drawings

A more complete appreciation of the invention and many of the attendant advantages thereof will be readily obtained as the same becomes better understood by reference to the following detailed description when considered in connection with the accompanying drawings, wherein:
FIGURE 1 shows cation exchange chromatographic purification of Limulus endotoxin binding protein using CM-Sepharose® resin. Peak 1: flow through, Peak 2: 0.25M NaCl elution, Peak 3: 1 M NaCl elution.
FIGURE 2 shows reversed phase chromatographic purification of Peak 3 from Fig. 1. Peak A: 25% isopropanol (IPA) elution, Peak B: 35% IPA elution, Peak C: 50% IPA elution, Peak D: column wash with 100% IPA.
FIGURE 3 is a plot of apparent endotoxin concentration (measured by LAL) versus protein concentration. Such a plot can be used to assess protein specific endotoxin inactivating activity. Specific activity is expressed as the amount of protein needed to achieve 50% reduction of endotoxin in the assay system (5 nanograms of endotoxin per 100 microliters). For convenience, units are expressed as micrograms⁻¹ protein x 10⁵ needed to reduce 5 nanograms/100 microliters of standard endotoxin by 50%.

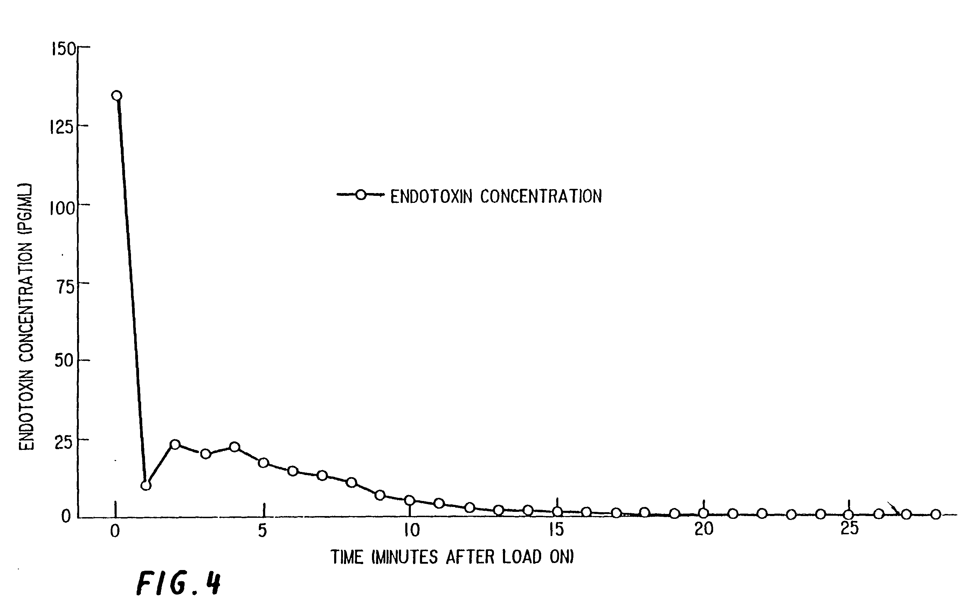

FIGURE 4 shows depyrogenation by affinity chromatography with endotoxin binding protein immobilized to a solid support.

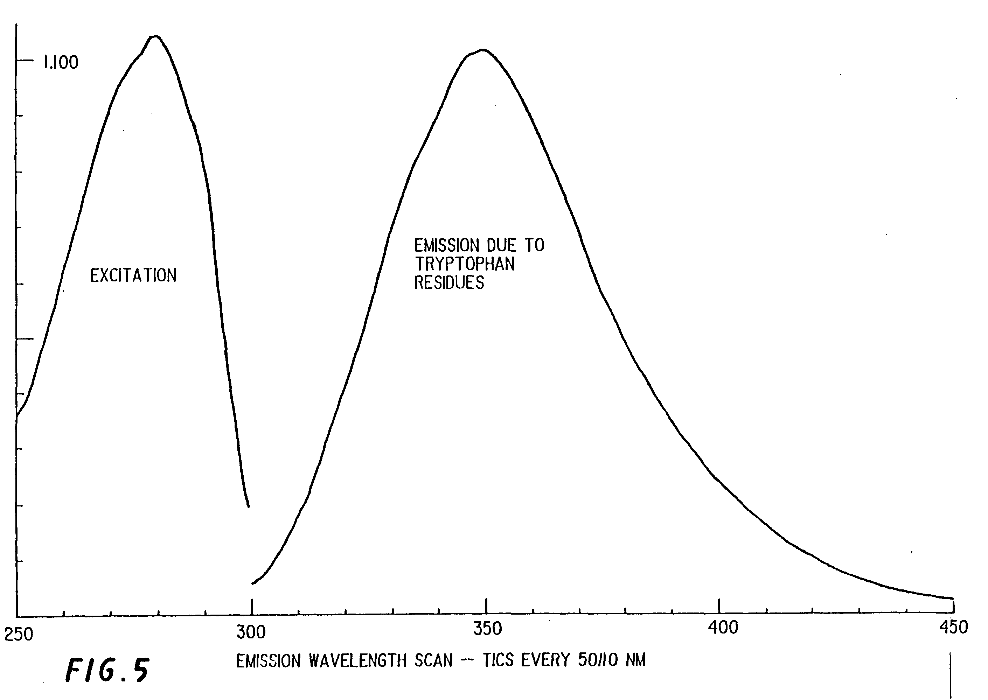

FIGURE 5 shows fluorescence excitation and emission spectra of Limulus endotoxin binding protein.
FIGURE 6 shows successive decreases in the emission spectra of Limulus endotoxin binding protein upon successive additions of endotoxin.
FIGURE 7 shows lack of successive decreases in the emission spectra of human serum albumin upon successive additions of endotoxin (negative control).
FIGURE 8 shows lack of successive decreases in the emission spectra of peak B, FIGURE 2 upon successive additions of endotoxin (negative control).
FIGURE 9 shows a fluorescence titration of Limulus endotoxin binding protein at pH 3.86. K_{D} = 2.41 micromolar.
FIGURE 10 shows fluorescence titration of Limulus endotoxin binding protein at pH 6.91. K_{D} = 0.51 micromolar.
FIGURE 11 shows fluorescence titration of Limulus endotoxin binding protein at pH 8.80. K_{D} = 2.40 micromolar.
FIGURE 12 shows the effect of pH on dissociation constant of Limulus endotoxin binding protein.

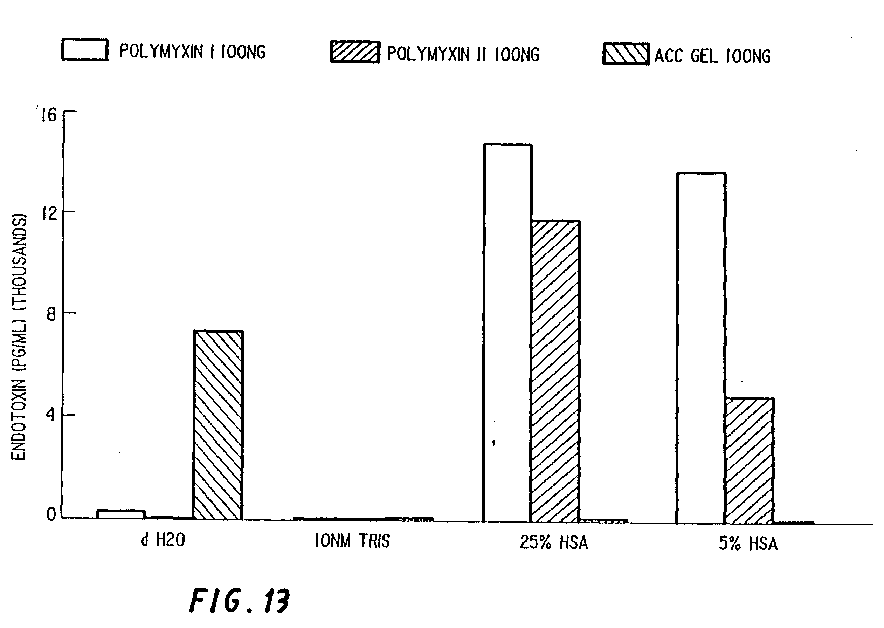

FIGURE 13 is a comparison of immobilized Limulus endotoxin binding protein compared to two immobilized polymyxin resins in proteinaceous and non-proteinaceous solutions.

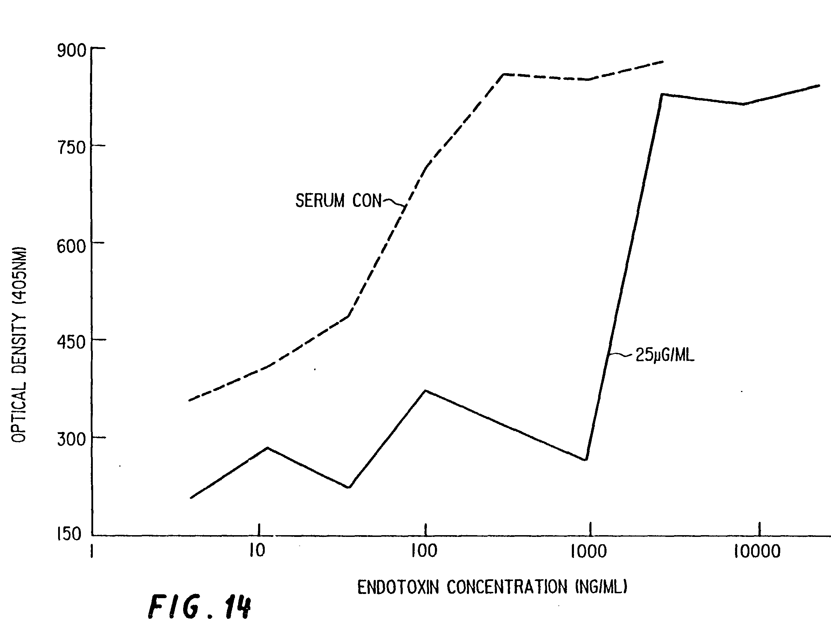

FIGURE 14. Demonstration of activity of Limulus endotoxin binding protein in the presence of total human serum. The dashed line represents serum alone as a control (68 nanograms of endotoxin per ml inactivated). The solid line represents serum plus 25 micrograms/ml added endotoxin binding protein (1600 nanograms endotoxin per ml inactivated).

### Best Mode For Carrying Out the Invention

The endotoxin binding protein may be isolated from any horseshoe crab. For example, any of the four known species of horseshoe crabs could be used. These species are:
Limulus polyphemus
Tachypleus gigas
Tachypleus tridentatus
Carcinoscorpius rotundicauda
Especially preferred among these is Limulus polyphemus, the horseshoe crab which is found along the North American coast. The endotoxin binding protein may be isolated by a procedure which is summarized hereinbelow. The procedure is illustrated for the Limulus horseshoe crab, but these procedures may be applied to any known horseshoe crab, as recited herein.

It was surprisingly found that the cellular debris produced during lysate production from Limulus amebocytes contains significant amounts of the endotoxin binding protein. The cellular debris was found to have even more activity than lysate itself. By "cell debris" is meant the insoluble material remaining when Limulus amebocytes are lysed by hypotonic shock. It includes nuclei, cell debris and some lysate proteins. The majority is insoluble material. Hypotonic shock may be accomplished by treating the Limulus amebocytes with endotoxin-free distilled water, preferably at 4°C, with shaking (e.g., for 12 h). The mixture is centrifuged, separating the lysate (soluble supernatant) from the cell debris.

The next step is to extract from the cell debris the Limulus amebocyte binding protein. Many solvents have been tested, including water, several alcohols (e.g. methanol, ethanol, isopropanol, and butanol), acetone, chloroform, acetonitrile, acids (e.g. HCl and H₂SO₄), bases (e.g. NaOH and ethanolamine), salts (e.g. NaCl), and detergents (e.g. Tween, triton X-100, and SDS). The best results were obtained with denaturants such as urea and guanidine hydrochloride. Surprisingly, six molar solutions of denaturants were effective in extracting the protein without affecting biological activity. This concentration of denaturant would be expected to inactivate most proteins. Thus, the first step in the purification procedure herein is extraction of cellular debris from Limulus amebocyte lysate with a denaturant to produce an extract. The concentration of the denaturant can range from 1 M to 10 M. A more preferred range would be 3 M to 8 M. A most preferred concentration is around 6 molar. Urea is the preferred denaturant, because it can be made free of endotoxin by ultrafiltration and it is not readily contaminated by endotoxin containing bacteria.

Preferably, prior to loading the extract onto a cation exchange column, an ultrafiltration step is performed. This step is also accomplished using urea or another denaturant as described above for extraction. In particular, the extract from the cell debris is crudely filtered with a filter aid such as diatomaceous earth (e.g. Celite, Manville Corp.) or one of the cationic or anionic polymeric particles in colloidal suspension (e.g. Biocryl, Supelco division of Rohm and Haas Corp.), then passed through an ultrafiltration membrane. Ultrafiltration membranes may be used in any known form (e.g., plain film, hollow fiber, tubular and spiral) and may be made of any material. Preferred materials are polysulfone, polyvinylidene fluoride, polyacrylonitrile, nylon, or cellulose. Most preferred is a polysulfone, flat or hollow fiber type ultrafiltration membrane. The preferred molecular weight cut-off of the ultrafiltration membrane is 20,000 daltons to 50,000 daltons as can be commercially obtained from Millipore, Filtron or other membrane filter manufacturers. The most preferred membrane has a 30,000 dalton cut-off. The Limulus endotoxin binding protein is now in the filtrate at a very low concentration. The filtrate from the first membrane is concentrated using a second ultrafiltration membrane having a molecular weight cut-off of 5,000 to 10,000 daltons, preferably an 8,000 dalton cut-off membrane. The second ultrafiltration membrane may be the same or different material as the first ultrafiltration membrane, and is preferably made of polysulfone. The principle of operation is the same as the 30,000 cut-off membrane, however, the endotoxin binding protein is now in the retentate with all other proteins greater than e.g. 8,000 daltons.

After the above step, the retentate is subjected to cation exchange chromatography using cellulose, cross-linked agarose or hydrophilic vinyl polymer resins derivatized with carboxymethyl (CM), Sulphopropyl (SP), Sulphonate (S), or other anionic group. These can be obtained from several manufacturers including Pharmacia (Sephadex® , Sepharose® ), Tosoh Corp. (Toyopearl® ) or BioRad (Cellex® , Bio-Gel). The most preferred resin is CM-Sepharose® . The pH of loading and elution buffers can range from 4 to 8, with the most preferred range of pH 5 to 5.5. In this step also, urea (or another denaturant as described above for extraction) is an important constituent. All column elution buffers must contain urea (at least about 3 molar) to elute the endotoxin binding protein cleanly from the column.

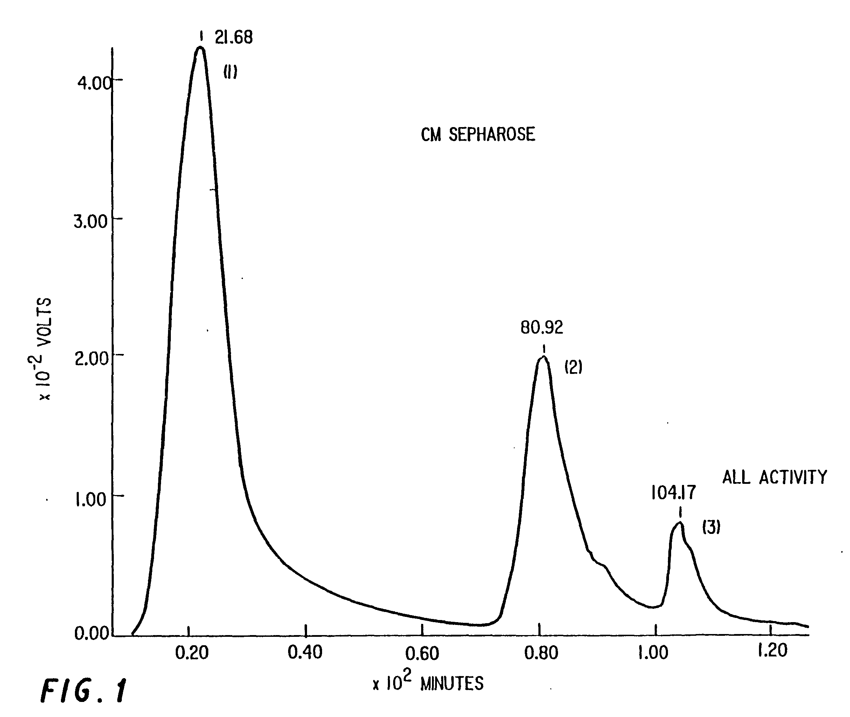

The elution from the column is accomplished by a step gradient of a salt such as ammonium chloride, potassium chloride or sodium chloride. Sodium chloride is preferred. FIGURE 1 shows the results of the CM Sepharose® chromatographic step. The preferred concentration of urea in the eluent used in this step is 1 M to 6 M. A preferred concentration of urea in this step is 2 M to 4 M. A most preferred concentration is 2.5 M to 3.5 M. When sodium chloride is used in the eluant, biological activity elutes at a concentration of sodium chloride of from around 0.5 to 1 molar.

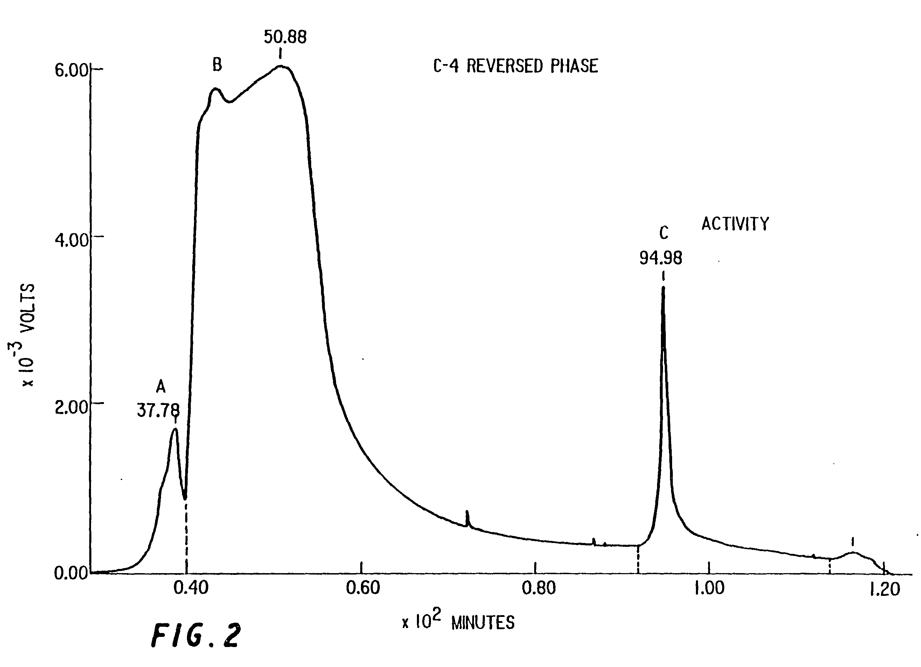

After the cation exchange step, the salt-eluted peak of activity is applied to a reversed-phase column. The preferred method employs a resin having 4, 8, or 18 carbon chains (C-4, C-8 or C-18, respectively). The most preferred method employs a C-4 resin commercially available from such resin manufacturers as Vydac, Waters, Tosoh Corp., etc. The protein is eluted by a step gradient of, for example, isopropanol and trifluoroacetic acid (TFA). The concentration of trifluoroacetic acid is ideally 0.2%, but can range from 0 to 0.4%, preferably 0.15 to 0.25%. FIGURE 2 shows the location of the activity during treatment on the reversed-phase column. The reversed-phase column step effects desalting and further purifies the material to virtual homogeneity. The Limulus protein is remarkably stable to the organic solvents and low pH of this column. The protein is stable over a pH range of 1-3 in the presence of TFA. The material may now be lyophilized. The purified Limulus protein has an isoelectric pH (pI), which is greater than 10, indicating a very basic protein. The molecular weight of the protein as determined by SDS-PAGE is 12,500 ± 100. The first three N-terminal amino acids of this purified material are
Asp-Gly-Ile

It should be noted that Watson and Sullivan, U.S. Patent 4,107,077, teaches that there is an increase in sensitivity of the lysate by organic extraction with chloroform. It appeared logical that a protein which inactivated endotoxin would appear as an inhibitor in the endotoxin assay. Thus, it was hoped that the protein was the inhibitor which is extracted into chloroform and could be recovered from that extract. However, surprisingly, this was not successful. Since the protein is fairly hydrophobic, it may be in the organic extract, but denatured in some way.

The majority of the purified material had an amino acid sequence which appears to be identical to a protein from Limulus that was isolated by Tanaka et al. The material was subsequently purified by Iwanaga et al, as reported in the International Symposium on Pyrogen, held June 23,-26, 1987 in China. However, in the latter case, a different purification procedure was employed.

In addition to a protein having an apparently identical amino acid sequence to that reported in Iwanaga et al, some related proteins were also purified, which have different amino acid sequences. These variant proteins have not previously been described. In a first protein, on the N-terminal thereof, a serine rather than an aspartic acid residue is located. Furthermore, an asparagine is located in the second position from the N-terminal rather than a glycine as reported in Iwanaga et al. Therefore, a protein having an initial amino acid sequence of
Ser-Asn-Ile-Trp-Thr-
is also part of the present invention. Other possible protein derivatives are those beginning with
Asp-Asn-
Ser-Gly-, and
Ser-Asn-, and also a protein having an N-terminal asparagine with one less amino acid than the natural sequence. Each of these variant proteins is also part of the present invention, and they may be used in each aspect of the invention described hereinbelow. They will be referred to as endotoxin binding protein variants (or variant proteins for short), as distinguished from the natural sequence endotoxin binding protein, which is the protein having the amino acid sequence reported in Iwanaga et al.

The variant proteins were not separated from each other. Rather, upon sequencing the most purified samples, some positions were uniformly one amino acid, while other positions showed major variation. This is interpreted as a mixture of proteins showing micro-heterogeneity. It is consistent with the existence of a gene family coding for these proteins whose individual gene sequences are very homologous, but not identical. It remains to be seen if their individual specific activities vary significantly.

Gene sequences which encode the polypeptides, and vectors containing these gene sequences, can be used to transform microorganisms such as E. coli, yeast, etc. The transformed microorganisms can be used to produce large quantities of the polypeptide materials, including the variant proteins described above. The genes may be altered so as to maximise codon expression in a given host. Procedure for Assaying for Endotoxin Binding Activity

To follow the purification of the endotoxin binding protein during the above purification steps, assays can be performed in solution. One publication describing typical assays for LR₅₀ values is Novitsky et al, J. Clin. Microbiol. pp. 211-216 (1985). A specific procedure follows:

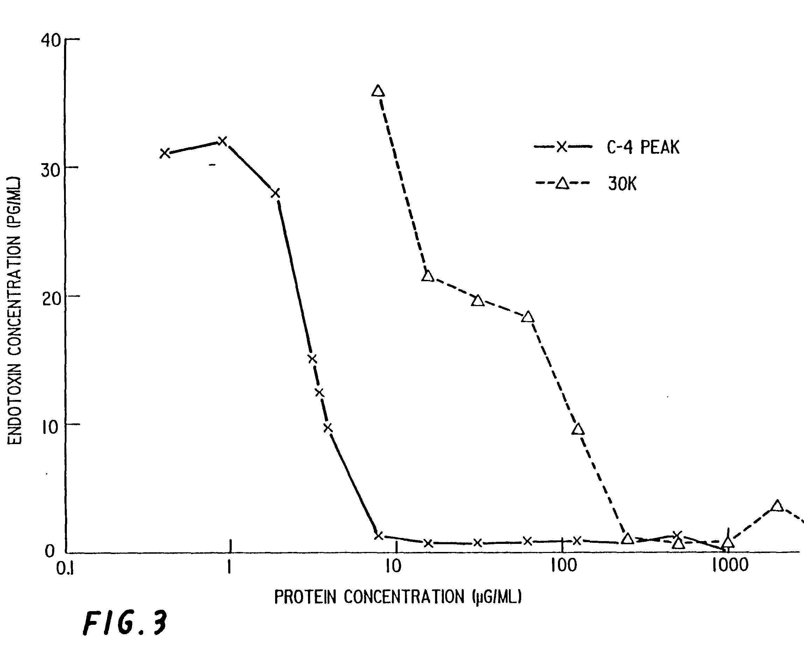

A 96-well microtiter plate is used. Protein fractions are serially diluted across the top row and mixed with a standard endotoxin solution. There is another series of dilutions done on all samples to get within range of the assay, but basically, the endotoxin recovered in the well after mixing with a suspected endotoxin inactivating protein is measured. This is compared to negative controls and an endotoxin standard curve. It is found that the added endotoxin is inactivated at the high protein concentrations. As the protein is diluted across the plate, a point is reached where it is too dilute to inactivate the added endotoxin. This dilution is a measure of the protein specific activity. FIGURE 3 represents a graphical form of such experiments. In FIGURE 3, the dotted curve represents the crude extract after ultrafiltration, and the solid line is the most highly purified fraction from the reversed-phase column.

### Assay for Binding Affinity and Application of the Endotoxin Binding Protein in an Alternative Endotoxin Assay

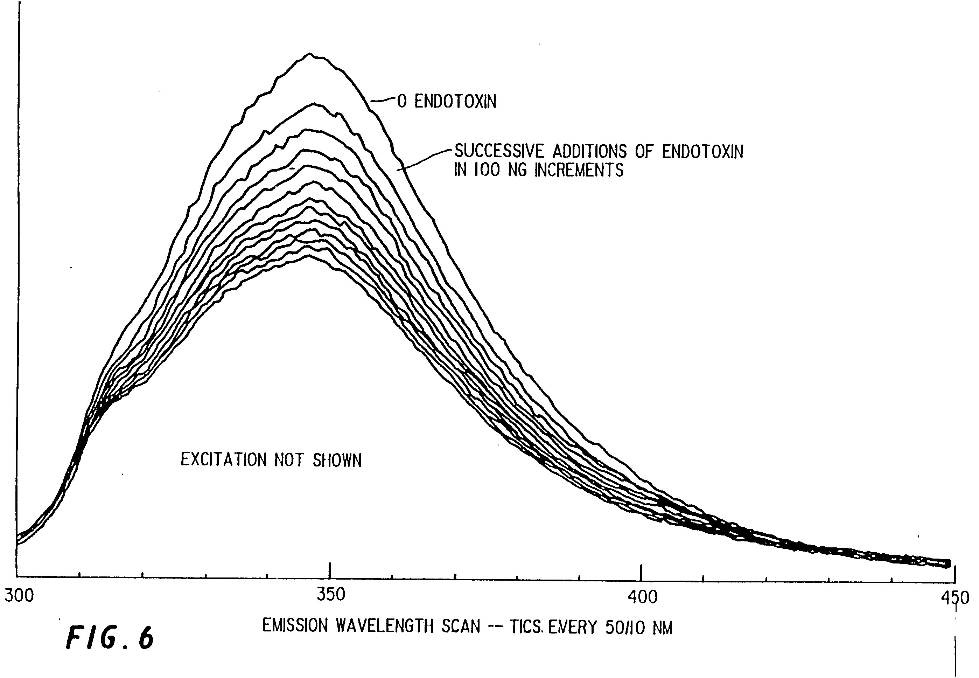

A known optical property of all proteins is the ability of some aromatic amino acids to fluoresce when excited at certain wavelengths. The present inventors looked specifically at tryptophan residues. These are excited at around 283 nm and fluoresce at around 350 nm. See FIGURE 6.

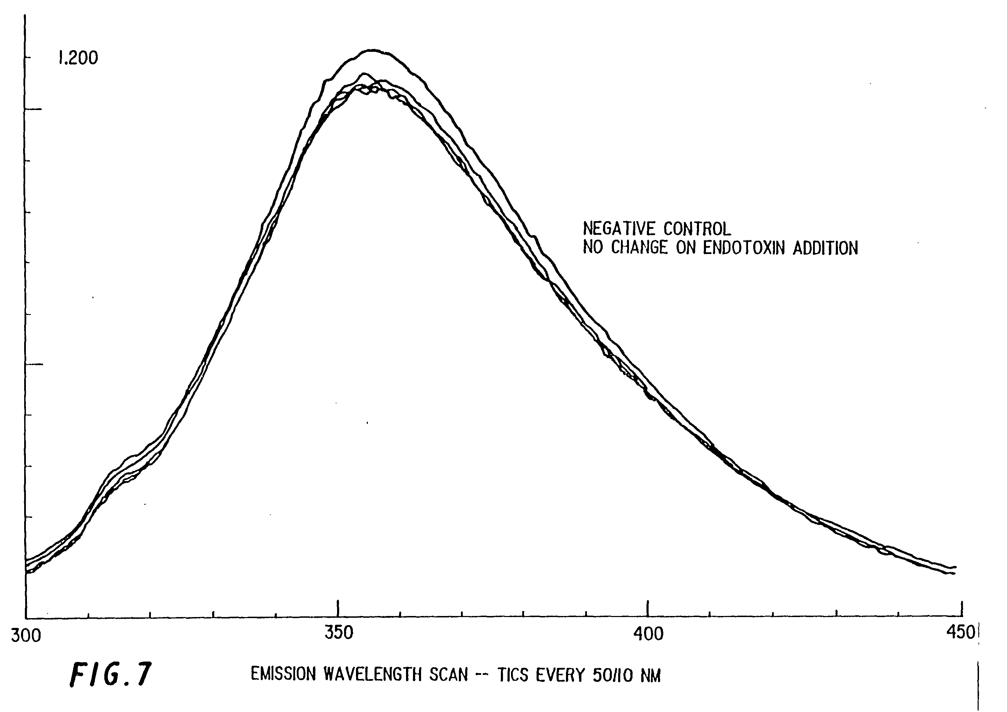

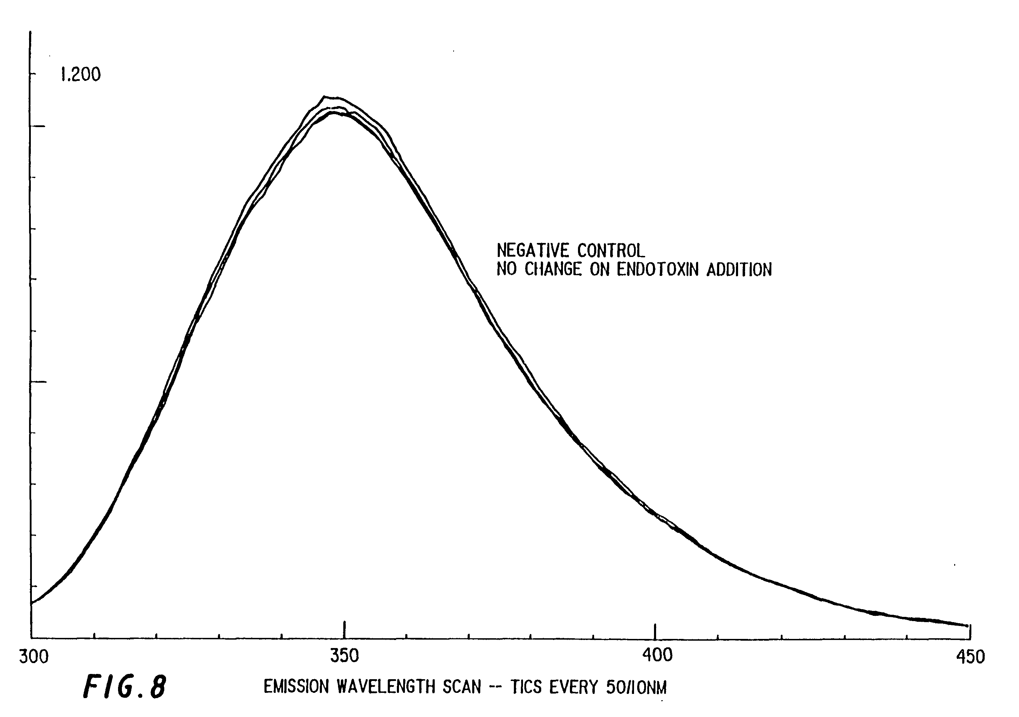

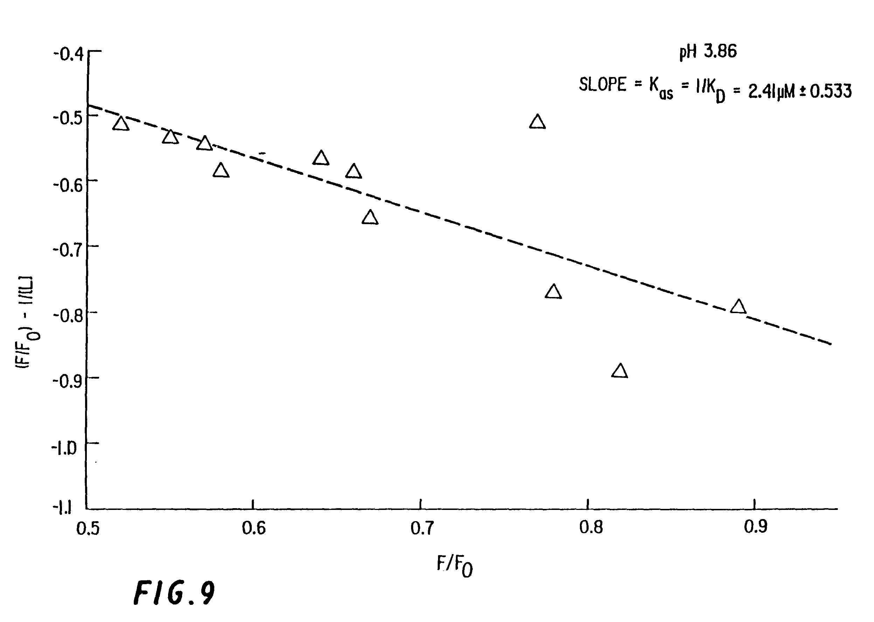

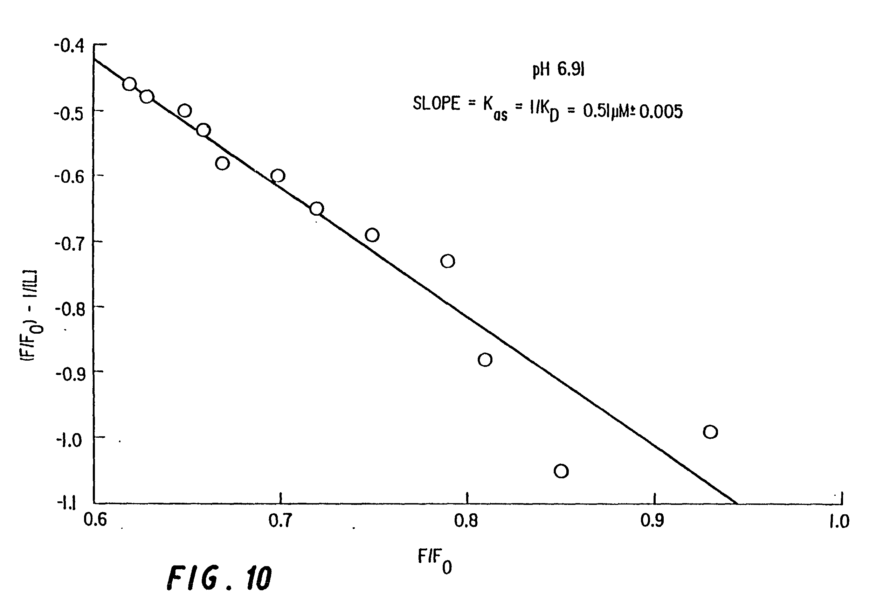

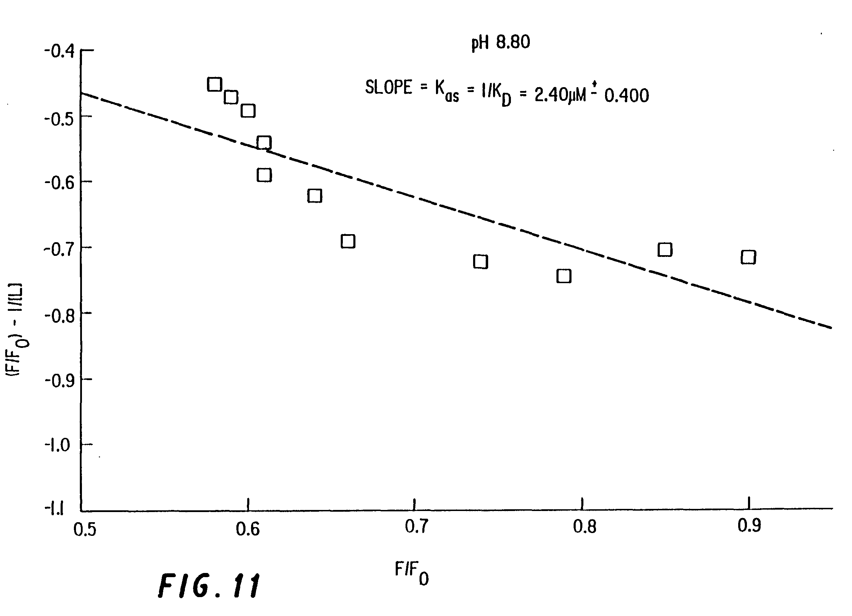

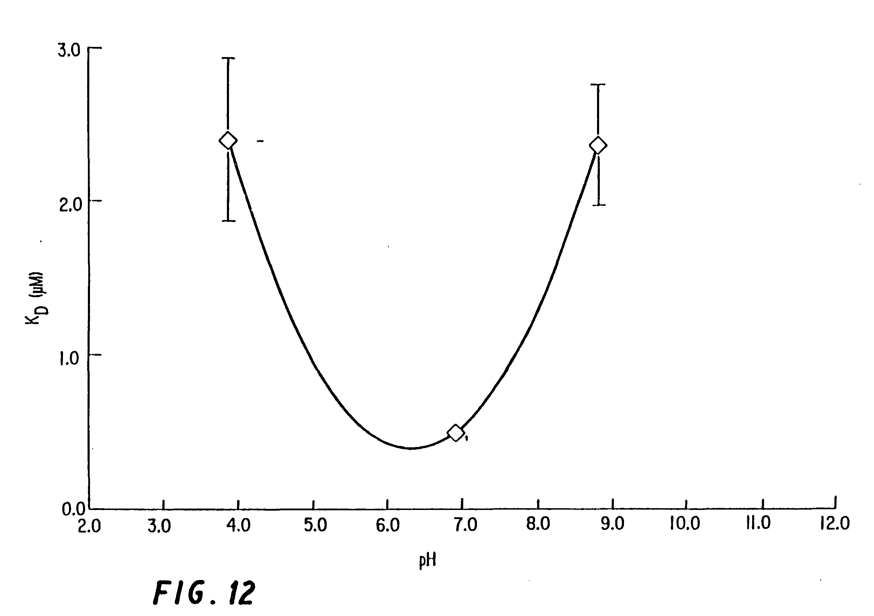

The inventors examined a fluorescence maxima at 350 nm of the endotoxin binding protein at various concentrations of endotoxin. There was a proportional quenching of that fluorescence, indicative of a tight association. See FIGURE 7. Other proteins as negative controls showed no such proportional change. See FIGURES 8 and 9. From this data, the inventors have been able to calculate the binding affinity constants between the present protein and endotoxin. See FIGURES 9-12.

The fluorescence quenching experiment may serve as the basis for an assay for endotoxin based on the physical change of a single protein upon exposure to endotoxin. The inventors have found that the quenching effect observed upon addition of endotoxin to the endotoxin binding protein does not appear to experience saturation up to a high concentration level of added endotoxin. In other words, although one would expect the relationship between concentration of endotoxin and quenching to be a nonlinear one, the linearity of the relationship extends over a remarkably wide range of concentration of endotoxin. This unexpected aspect of the present assay enables the assay to be used over a wider range or endotoxin concentrations than could have been expected. Ranges of the ratio of endotoxin binding protein to endotoxin that are expected to be effective are 0.1:1 to 5000:1, preferably 1:1 to 2000:1, most preferably 10:1 to 1000:1.

In another embodiment, the present invention relates to biosensors. Biosensors rely on a specific binding ligand immobilised on a solid (e.g. quartz) chip. Electrical potential between two electrodes, or the acoustic wave perturbation between two electrodes is measured.

The current method for measuring endotoxin is the Limulus amebocyte lysate assay. This involves a cascade of enzymes which are activated by endotoxin and result in the formation of a gel, analogous to a mammalian blood clot. Since multiple enzymes are involved, many substances interfere with the overall reaction, causing enhancement or inhibition. In addition, the key enzymes of the cascade are proteases so other proteolytic enzymes, such as trypsin, can cause gel formation. To eliminate these problems, samples containing interfering substances must be diluted beyond the point where they interfere. In many cases the dilution factor can be hundreds or thousands. The net effect is to decrease the sensitivity in measuring absolute endotoxin concentrations in those samples.

One of the promising potentials that a single endotoxin binding protein has is to eliminate the multiple interfering effects seen in the LAL assay. Even if the sensitivity were one tenth that of LAL, the lack of a need to dilute would compensate, making the sensitivity equivalent. Biosensors take advantage of specific binding affinities of such molecules. Antibodies are an example which have been used. Bound to the surface of a silicon chip, they bind to their immunogen and cause a change in the surface which is measured electrically. Depending on the type of sensor, capacitance, resistance or acoustic wave chances are measurable. Depending on the concentrations and volumes of the samples, the time required for each measurement can be from seconds to minutes.

The electronic component of the biosensor could measure voltage (potentiometric), current (amperometric), light, sound, temperature, or mass (piezoelectric). The biosensors of the present invention can be based on technology which is known, such as described in Biotechnology 5, 437-440 (1987), and Transducers 1987, the abstract entitled "Development or a Surface Acoustic Wave Biosensor", and "Recent Progress in Biosensor Development", Hall, E. A. H., Int. J. Biochem. 1988, 20(4), 357-62, each of which is incorporated herein by reference. Biosensor information is also contained in U.S. Patents 4,721,677, 4,680,268, H 0,000,201, and 4,614,714, each of which is incorporated herein by reference.

An actual device employing a biosensor would be similar to a flow cell. The sample would enter, be exposed to the reactive surface and a measurement made. Depending on the chemistry of binding, the bound ligand could be washed off to regenerate the surface or left on to measure a cumulative response. This may be adapted to an in-line device to monitor endotoxin contaminating events.

Due to the compact nature of the electronics, a portable field unit is feasible. This could be used onsite for checking water purity or process cleanliness during pharmaceutical manufacture, kidney dialysis unit pyrogen checks, or any other application where the conventional LAL is used now. Since endotoxin is a component of gram negative bacterial cell walls, binding of whole bacteria should be measurable. An extension of this technology should make possible remote sensors to monitor water quality in the environment.

By coupling the binding protein to latex microspheres using a chemistry similar to that used to immobilize the protein on chromatographic resins or membranes, another method to quantitate endotoxin is constituted. By exposing latex microspheres coated with endotoxin binding protein (e.g. from Limulus) to endotoxin, the microspheres agglutinate. Such agglutination is commonly employed in assays based on antibodies to the specific ligand to be measured (Hechemy, K.E.; Michaelson, E.E. Laboratory Management 1984, 22(6):27,ff (Part I) and 22(7):26,ff (Part II); Babson, A.L., Opper, C.A. and Crane L.J. American Journal of Clinical Pathology (1982), 77(4):424-9). In such techniques, the agglutination phenomenon is thought to be due to crossiinking between multiple binding sites on the antibody molecule complexing with multiple sites on the antigen. Due to the high ratio of endotoxin binding protein to endotoxin needed to be effective in our other solution experiments, it is thought the number of endotoxin binding sites on the protein is one or less. By covalently coupling many molecules of binding protein per microsphere, a multiple binding site reagent is created which is now possible to function as a specific agglutinin. Alternatively, the binding protein can be linked to one suspension of beads and endotoxin or lipid A linked to a second suspension. On mixing, these two suspensions will agglutinate. This may also be employed as an assay for endotoxin in solution by the ability of the free endotoxin to inhibit such agglutination. In this case, agglutination is inversely proportional to the unknown endotoxin concentration.

The invention now being generally described, it will now be illustrated in greater detail by the following examples, which are presented herein for illustration only and are not intended to be limiting of the present invention, unless so indicated.

### Examples

### Example 1

### Assay in of in vitro Inactivated Endotoxin by Pyrogen Testing in Rabbits:

E. coli endotoxin was prepared at 100 nanograms per ml. in Phosphate Buffered Saline (PBS). This was mixed with 200 micrograms of Limulus Endotoxin Binding Protein and incubated at 37 degrees Celsius for one hour. Control solutions were prepared of endotoxin (100 nanograms/ml) only and PBS only. Three vials of each of the three solutions were prepared containing 2 ml/vial. 1.5 ml of each solution was injected intravenously into individual 3 kilogram rabbits and their body temperatures measured continuously for six hours with data points recorded every 10 minutes. The final dose of endotoxin or inactivated endotoxin was 50 nanograms/kilogram.

In rabbits, a 5 nanograms/kilogram dose of endotoxin elicits a measurable fever response, showing a peak at one hour after injection. The present results showed the rabbits receiving only endotoxin at 50 nanograms/kilogram did indeed develop a body temperature elevated 1.64 (S.D. 0.236) degrees Celsius. Those rabbits receiving only PBS or endotoxin inactivated with Limulus Endotoxin Binding Protein maintained a normal body temperature.

### Example 2

### Assay of In vivo Prophylactic Efficacy of Limulus Endotoxin Binding Protein:

Limulus Endotoxin Binding Protein is injected intravenously into rabbits at two dose levels, 5 micrograms/kilogram and 50 micrograms/kilogram. Control animals receive injections of PBS. Fifteen minutes later, all animals receive intravenous injections of 50 nanograms/kilogram E. coli endotoxin. Body temperature is monitored continuously over six hours and recorded every 10 minutes. The normal course of increase in temperature peaking at one hour after injection is seen in control animals preinjected with PBS only. Those animals receiving preinjection of endotoxin binding protein at 50 micrograms/kilogram showed an increase of 1.55 (S.D. 0.225) degrees Celsius. Animals receiving 5 micrograms/ kilogram demonstrated temperature increases of 1.85 (S.D. 0.270).

### Example 3

### Assay of In Vivo Therapeutic Efficacy of Limulus Endotoxin Binding Protein:

E. coli endotoxin is injected intravenously into 9 rabbits at a dose of 50 nanograms/kg. After 15 minutes, three were injected intravenously with 5 micrograms Limulus Endotoxin Binding Protein, three were injected intravenously with 50 micrograms Limulus Endotoxin Binding Protein and three received PBS. Volumes of all injections were 0.5 ml/kg. Body temperatures are monitored for 6 hours and data collected every 10 minutes. Animals receiving endotoxin and the PBS only, manifest the normal peak fever response one hour after toxin administration. Those animals receiving therapeutic injections of the Limulus protein exhibit a much reduced fever response proportional to the amount of protein which is administered.

### Example 4

### Endotoxin Inactivating Potential of Limulus Endotoxin Binding Protein in Human Serum:

In order to assay the potential effectiveness of the endotoxin binding protein as a therapeutic or prophylactic agent for septic shock or related disorders in humans, the protein was tested for its ability to inactivate endotoxin in the presence of whole human serum. The assay was conducted in a 96-well tissue culture multiwell plates as described in detail in Novitsky, et al., J. Clin. Micro., 20:211-216 (1985). To each well were added in order 0.05 ml of serum only or serum with 25 micrograms/ml Limulus Endotoxin Binding Protein, 0.05 ml E. coli endotoxin solution. The plates were covered with Parafilm to prevent evaporation, agitated on a mechanical vibrating platform for 15 seconds and incubated at 37 degrees Celsius. Multiwell plates were then uncovered, and 0.1 ml of LAL was added to each well. The plates were subsequently handled and read as described for the LAL endotoxin assay. Serum with the added endotoxin binding protein was able to inactivate increasingly larger amounts of endotoxin (2,000 nanograms/ml) while serum alone was able to bind and/or inactivate only 80 nanograms/ml.

### Example 5

### Universality of Endotoxin Type Inactivated by the Limulus Endotoxin Binding Protein.

In order to investigate the mechanism of the endotoxin binding phenomenon and determine the breadth of endotoxin types against which the binding protein is effective, endotoxin from several species of Gram negative bacteria as well as lipid A. were tested. These included endotoxin from Klebsiella pneumoniae, Serratia marcescens, Salmonella enteritidis, Eschericia coli 0113 wild type, E. coli rough mutant (J-5), Salmonella abortus equi, and Lipid A from S. minnesota Re 595.

The endotoxin binding protein was mixed with the endotoxins or lipid A at a ratio of 5:1 to 1,000:1 in the presence of 10 millimolar Tris buffer, pH 6-8. In all cases the measurable endotoxin after mixing was reduced 85% to 99.5%.

The invention now being fully described, it will be appreciated that the invention may be practiced otherwise than as specifically set forth herein.

## Claims

1. A method for assaying endotoxin concentration, which comprises contacting serial aqueous dilutions of material suspected of containing endotoxin with a known quantity of endotoxin binding protein,
observing the fluorescence emission at at least one wavelength of the endotoxin binding protein before and after contact with aqueous dilutions of said material suspected of containing endotoxin,
correlating the levels of fluorescence emission with at least one known emission level to thereby determine the quantity of endotoxin present in said material suspected of containing endotoxin.

2. The method of claim 1, wherein said material is a body fluid.

3. The method of claim 1 or claim 2, wherein said fluorescence emission of the endotoxin binding protein is measured at from 340 to 360 nanometers.

4. The method according to claim 1 or claim 2, wherein said fluorescence emission is produced by excitation at from about 275 to 295 nanometers.

## Patentansprüche

1. Verfahren zum Assay der Endotoxinkonzentration, das das Kontaktieren wässeriger Reihenverdünnungen eines Materials, von dem angenommen wird, daß es Endotoxin mit einer bekannten Quantität eines Endotoxinbindungsproteins enthält,
das Beobachten der Fluoreszenzemission bei zumindest einer Wellenlänge des Endotoxinbindungsproteins vor und nach dem Kontakt mit den wässerigen Verdünnungen des besagten Materials, von dem angenommen wird, daß es Endotoxin enthält,
das Korrelieren der Niveaus der Fluoreszenzemission mit zumindest einem bekannten Emissionsniveau, um dadurch die Quantität des Endotoxins, das in besagtem Material vorliegt, von dem genommen wird, daß es Endotoxin enthält, zu bestimmen, umfaßt.

2. Verfahren nach Anspruch 1, wobei besagtes Material eine Körperflüssigkeit ist.

3. Verfahren nach Anspruch 1 oder Anspruch 2, wobei besagte Fluoreszenzemission des Endotoxinbindungsproteins bei 340 bis 360 Nanometern gemessen wird.

4. Verfahren nach Anspruch 1 oder Anspruch 2, wobei besagte Fluoreszenzemission durch Anregung bei etwa 275 bis 295 Nanometern erzeugt wird.

## Revendications

1. Procédé pour tester la concentration d'endotoxine, qui comprend la mise en contact de dilutions aqueuses en série d'un matériau suspecté de contenir l'endotoxine avec une quantité connue de protéine de liaison de l'endotoxine,
l'observation de l'émission de fluorescence au moins à une longueur d'onde de la protéine de liaison de l'endotoxine avant et après contact avec les dilutions aqueuses dudit matériau suspecté de contenir l'endotoxine,
la corrélation des taux d'émission de fluorescence avec au moins un taux d'émission connu pour déterminer de cette façon la quantité d'endotoxine présente dans ledit matériau suspecté de contenir l'endotoxine.

2. Procédé selon la revendication 1, dans lequel ledit matériau est un fluide corporel.

3. Procédé selon la revendication 1 ou la revendication 2, dans lequel ladite émission de fluorescence de la protéine de liaison de l'endotoxine est mesurée entre 340 à 360 nanomètres.

4. Procédé selon la revendication 1 ou la revendication 2, dans lequel ladite émission de fluorescence est produite par excitation à environ 275 à 295 nanomètres.
